# EUROPEAN PATENT APPLICATION

(11) **EP 2 684 573 A2**
(43) Date of publication of application: **15.01.2014**
(21) Application number: 13186756.6
(22) Date of filing: 12.12.2008
(51) Int. Cl.: A61K 41/00, A61P 35/00, A61K 49/00, A61K 9/02, A61K 9/28, A61K 9/48, A61K 31/197, A61K 9/00, A61K 9/16, A61K 9/20, A61K 9/50

(54) **Use of 5-aminolevulinic acid and derivatives in a solid form for photodynamic treatment and diagnosis**

(30) Priority: 12.12.2007 GB 0724279
(62) Divisional of application: 08860052.3
(71) Applicant: Photocure ASA, 0275 Oslo (NO)
(72) Inventor: Klaveness Jo, N-1166 Oslo (NO); Klem, Bjorn, N-0286 Oslo (NO); Stensrud, Gry, N-1484 Nittedal (NO); Godal, Aslak, N-0372 Oslo (NO); Brænden, Jon Erik, N-0491 Oslo (NO)
(74) Representative: Golding, Louise Ann

(57) **Abstract**

The present invention relates to a solid pharmaceutical product provided in the form of a suppository which comprises a photosensitiser which is a 5-ALA ester of general formula I, or a pharmaceutically acceptable salt thereof:

R²₂N-CH₂COCH₂-CH₂CO-OR¹ (I)

(wherein
R¹ represents a substituted or unsubstituted, straight-chained, branched or cyclic alkyl group; and
each R² independently represents a hydrogen atom);
and at least one pharmaceutically acceptable carrier or excipient which is a hard fat or a mixture of hard fat and additives.

The pharmaceutical product may be used in the photodynamic treatment of cancer, and infections associated with cancer, and in the treatment of non-cancerous conditions, in particular in treating cancerous or non-cancerous conditions in the female reproductive system, for example cervical cancer and infections caused by the human papillomavirus.

## Description

This invention relates to methods of photodynamic treatment and diagnosis of conditions such as cancer, and in particular to the use of solid pharmaceutical products comprising a photosensitiser which is 5-aminolevulinic acid (5-ALA) or a precursor or derivative thereof (e.g. a 5-ALA ester) in such methods. The pharmaceutical products which are described herein are particularly suited to use in the treatment or diagnosis of cancer and non-cancerous conditions in the lower part of the gastrointestinal system (especially in the lower small intestine, colon and rectum) and in the female reproductive organ system (i.e. uterus, cervix, vagina).

Photodynamic therapy (PDT) is a relatively new technique that has been used in the treatment of various cancers as well as other diseases. PDT involves the administration of photosensitizing agents followed by exposure to photoactivating light in order to activate the photosensitizing agents and convert them into cytotoxic form resulting in the destruction of cells and thus treatment of the disease. Several photosensitizing agents are known and described in the literature including 5-aminolevulinic acid (5-ALA) and certain derivatives thereof, e.g. 5-ALA esters.

Currently three pharmaceutical products comprising 5-ALA or an ester thereof are in clinical use for PDT and photodynamic diagnosis (PDD). These are Metvix® and Hexvix® both developed by Photocure ASA (Oslo, Norway) and Levulan Kerastick® developed by DUSA Pharmaceuticals (Canada). Metvix® is a dermal product for treatment of actinic keratosis and basal cell carcinoma which comprises methyl ALA ester in an emulsion (cream). Hexvix® is an aqueous solution which comprises hexyl ALA ester for instillation into the urine bladder for diagnosis of bladder cancer. Levulan Kerastick® is a 2-compartment formulation that is used to prepare a solution of 5-ALA immediately before application. This product can be used for treatment of skin diseases.

Although these products are clinically useful, they all suffer from the disadvantage of instability of 5-ALA. 5-ALA and esters thereof are subject to a broad spectrum of decomposition reactions which limit the shelf life of pharmaceutical products in which they are present.

A number of different strategies have been adopted to try to overcome this problem. For instance, with the Metvix® product the problem of instability is addressed by storing the cream in cold conditions and with the Levulan Kerastick® product the 5-ALA is supplied separately from its diluent so the solution administered to the subject is only prepared immediately before use. Hexvix® is supplied as a lyophilised powder and dissolved in an aqueous solution immediately before use.

These approaches, however, have disadvantages. For example, it is not always convenient to transport and store medicines in cold conditions. Moreover it is also generally preferable to provide pharmaceutical compositions in a ready-to-use form as these are most convenient for medical practitioners. Provision of ready-to-use forms also enables the compositions to be prepared with a reliable and accurate concentration. This is particularly important in the treatment and diagnosis of the majority of diseases including cancer where it can be critical that the correct dosage of therapeutic is administered.

US 2003/125388 describes an alternative approach to provision of stable 5-ALA formulations wherein 5-ALA or a derivative thereof is dissolved or dispersed in a non-aqueous liquid having a dielectric constant of less than 80 at 25°C which acts a stabiliser. It is hypothesised that the use of the non-aqueous liquid facilitates formation of the enol form of 5-ALA that then prevents its degradation. Examples of suitable non-aqueous liquids mentioned in US 2003/125388 include alcohols, ethers, esters, poly(alkylene glycols), phospholipids, DMSO, N-vinylpyrrolidone and N,N-dimethyl acetamide. This composition may form part of a kit for therapeutic or diagnostic use. The other part of the kit is a composition comprising water. In this case the two parts of the kit are mixed prior to use.

The approach in US 2003/125388 therefore suffers the same disadvantage as the Levulan Kerastick® in that it is generally undesirable to provide therapeutics in a form that requires the medical practitioner to formulate the pharmaceutical product that is actually administered. Moreover it may not always be desirable to administer a non-aqueous liquid to an animal.

A further disadvantage suffered by all of the above-mentioned strategies is that liquid and cream formulations are difficult to use for treatment, especially topical treatment, of a number of areas of the body. This is particularly disadvantageous in the case of cancer treatment since cancer occurs throughout the body.

Areas of the body which are difficult to treat using conventional PDT or PDD methods include the lower part of the gastrointestinal system and the female reproductive system (i.e. uterus, cervix and vagina). Currently, there are no products available for clinical use in photodynamic diagnosis or therapy of these parts of the body. This remains a significant problem, especially in relation to the colon and rectum which may be associated with several serious and life-threatening diseases like colitis, colorectal cancer, Crohn's disease, irritable bowel disease and various local infections, and in relation to the cervix which may be associated with cervical infections and cervical cancer. There remains a medical need for methods for earlier diagnosis of these diseases, especially colorectal cancer and cervical cancer.

Current diagnostic methods for colorectal cancer include monitoring of clinical symptoms like blood in the stools, lower abdominal pain or weight loss, coloscopy and X-ray based imaging methods. The prognosis of patients with colorectal cancer depends, as with most other cancer forms, on disease stage at the time of diagnosis and especially on whether the patient has developed distant metastasis. There are several therapeutic drugs in clinical use today for treating colorectal cancer, however, current drugs have their clinical limitations and there remains a medical need for further therapeutic regimes and alternative methods of early diagnosis.

One of the most serious infections of the cervix is human papilloma virus (HPV) which can develop into cervical cancer. HPV infection is a common factor in the development of almost all cervical cancer cases. Estimates for the prevalence of HPV infections vary, but can typically be around 30% in all women. Recently, HPV vaccines have been developed such as Gardasil® and Cervarix®. However, cervical cancer remains a life-threatening disease. The cancer is unfortunately often diagnosed late since symptoms may be absent until the cancer has developed to a late stage. One possible early sign of cervical cancer is vaginal bleeding. Cervical cancer is diagnosed based on biopsy procedures. The main treatment is surgery, however, radiation and chemotherapy can be used in late stages of the disease. The prognosis of patients with cervical cancer depends on disease stage at the time of diagnosis .

Oral formulations comprising 5-ALA and derivatives thereof, such as solutions, suspensions, classical tablets and capsules (containing aqueous formulations) have several disadvantages when used for the diagnosis and/or therapeutic treatment of cancer and non-cancerous diseases in the lower part of the gastrointestinal tract. These relate to shelf life stability of the pharmaceutical product, *in vivo* stability of the product during its passage through the whole gastrointestinal system, and systemic toxicity as result of absorption of 5-ALA or derivatives thereof. Systemic absorption in turn results in a reduction in clinical efficacy at the desired treatment site.

A need still therefore exists for alternative methods for photodynamic treatment and/or diagnosis of conditions such as, for example, cancer. In particular, a need exists for improved methods for the diagnosis and/or treatment of cancer and non-cancerous lesions in the lower part of the gastrointestinal system, especially conditions in the lower small intestine, the colon and rectum. A need also exists for improved methods for the diagnosis and/or treatment of cancer and non-cancerous lesions in the female reproductive organ system (i.e. uterus, cervix and vagina), especially the cervix.

It has now surprisingly been found that certain solid pharmaceutical products comprising 5-ALA or a derivative thereof (e.g. an ALA ester) overcome these problems of the prior art. The solid pharmaceutical products have stability at room temperature, are easy to handle and convenient to use, and can also readily be delivered to the lower part of the gastrointestinal system, especially to the lowest part of the small intestine, the entire colon and rectum. These can also readily be delivered locally to the female reproductive system, especially the cervix. Such products also generally address the problem of reduced efficacy of known formulations when treating these areas of the body. More specifically, these are capable of providing an effective concentration of 5-ALA or derivatives thereof at the desired treatment site (e.g. in the lower part of the gastrointestinal tract or the female reproductive system). These may also provide a substantially homogenous (i.e. uniform) distribution of the active photosensitizing agent at the intended site thereby further improving PDT or PDD treatment.

Thus, viewed from one aspect the invention provides the use of a photosensitiser which is 5-ALA or a precursor or derivative thereof (e.g. an ALA ester), in the manufacture of a pharmaceutical product for use in the photodynamic treatment or diagnosis (e.g. treatment) of cancer, an infection associated with cancer, or in the treatment or diagnosis of a non-cancerous condition, wherein said pharmaceutical product is in the form of a solid. Preferably, the product is for use in the photodynamic treatment or diagnosis of a cancerous or non-cancerous condition in the lower part of the gastrointestinal system or in the female reproductive system.

In a further aspect the invention provides the use of a photosensitiser which is 5-ALA or a precursor or derivative thereof (e.g. an ALA ester), in the manufacture of a pharmaceutical product for use in the photodynamic treatment of cancer in the lower part of the gastrointestinal system, wherein said pharmaceutical product is in the form of a solid.

In a yet further aspect the invention provides the use of a photosensitiser which is 5-ALA or a precursor or derivative thereof (e.g. an ALA ester), in the manufacture of a pharmaceutical product for use in the photodynamic diagnosis of cancer in the lower part of the gastrointestinal system, wherein said pharmaceutical product is in the form of a solid.

In a still further aspect the invention provides the use of a photosensitiser which is 5-ALA or a precursor or derivative thereof (e.g. an ALA ester), in the manufacture of a pharmaceutical product for use in the photodynamic diagnosis of a non-cancerous condition in the lower part of the gastrointestinal system, wherein said pharmaceutical product is in the form of a solid.

In a yet still further aspect the invention provides the use of a photosensitiser which is 5-ALA or a precursor or derivative thereof (e.g. an ALA ester), in the manufacture of a pharmaceutical product for use in the photodynamic treatment of a non-cancerous condition in the lower part of the gastrointestinal system, wherein said pharmaceutical product is in the form of a solid.

In an alternative aspect the invention provides the use of a photosensitiser which is 5-ALA or a precursor or derivative thereof (e.g. an ALA ester), in the manufacture of a pharmaceutical product for use in the photodynamic treatment of cancer in the female reproductive system (e.g. cervical cancer), wherein said pharmaceutical product is in the form of a solid.

In a yet further aspect the invention provides the use of a photosensitiser which is 5-ALA or a precursor or derivative thereof (e.g. an ALA ester), in the manufacture of a pharmaceutical product for use in the photodynamic diagnosis of cancer in the female reproductive system (e.g. cervical cancer), wherein said pharmaceutical product is in the form of a solid.

In a still further aspect the invention provides the use of a photosensitiser which is 5-ALA or a precursor or derivative thereof (e.g. an ALA ester), in the manufacture of a pharmaceutical product for use in the photodynamic diagnosis of a non-cancerous condition in the female reproductive system, wherein said pharmaceutical product is in the form of a solid.

In a yet still further aspect the invention provides the use of a photosensitiser which is 5-ALA or a precursor or derivative thereof (e.g. an ALA ester), in the manufacture of a pharmaceutical product for use in the photodynamic treatment of a non-cancerous condition in the female reproductive system, wherein said pharmaceutical product is in the form of a solid.

The diagnostic methods described herein may also be performed during surgery in which the diagnostic agent is given to the patient and surgery is then performed under blue light. The fact that the lesion or disease fluoresce under blue light aids the surgeon in defining the "surgical border" and thereby enables a more selective resection of the diseased area (e.g. tumor) to be performed. Use of the photosensitising agents herein described in methods of surgery forms a further aspect of the invention.

The therapeutic and diagnostic methods herein described may also be used in the form of a combined therapy. For example, a course of PDT performed in relation to a cancerous or non-cancerous condition using any of the methods herein described may be followed by a PDD method (e.g. to determine the extent to which PDT has been effective and/or to detect any re-occurrence of the condition).

In a further aspect the invention thus provides the use of a photosensitiser which is 5-ALA or a precursor or derivative thereof (e.g. an ALA ester), in the manufacture of a pharmaceutical product which is in the form of a solid for use in a method which comprises the steps of: (i) conducting photodynamic treatment of cancer or a non-cancerous condition in the lower part of the gastrointestinal system or in the female reproductive system of a patient; and (ii) conducting photodynamic diagnosis on said patient. At least one of steps (i) and (ii) is performed following administration to said patient of a photosensitiser which is 5-ALA or a precursor or derivative thereof (e.g. an ALA ester). Preferably, steps (i) and (ii) will both be performed following administration of such a photosensitiser.

In a still further aspect the invention provides a method of photodynamic treatment or diagnosis of cancer, an infection associated with cancer, or a non-cancerous condition, said method comprising the steps of:
(a) administering to a body a pharmaceutical product as hereinbefore defined;
(b) optionally waiting for a time period necessary for the photosensitiser to achieve an effective tissue concentration at the desired site; and
(c) photoactivating the photosensitiser.

In a yet further aspect the invention provides a photodynamic method of diagnosing cancer, an infection associated with cancer, or a non-cancerous condition in an animal pre-administered with a pharmaceutical product as hereinbefore defined, said method comprising:
(i) optionally waiting for a time period necessary for the photosensitiser to achieve an effective tissue concentration at the desired site; and
(ii) photoactivating the photosenstiser.

In a still further aspect the invention provides a solid pharmaceutical product comprising a photosensitiser which is 5-ALA or a precursor or derivative thereof, and at least one pharmaceutically acceptable carrier or excipient, wherein said pharmaceutical product is a suppository, capsule, pellet, pessary or tablet. Preferably said pharmaceutical product is in the form of a suppository, a pellet or a tablet.

A solid pharmaceutical product as hereinbefore defined for use in medicine forms a yet further aspect of the invention.

As used herein the term "pharmaceutical product" refers to the entity that is actually administered to a subject.

As used herein the term "solid" refers to the physical state of the entity being described (i.e. as being a solid, rather than a liquid or gas). Liquids, solutions, gels, and creams are therefore not encompassed by this term. Representative examples of solid pharmaceutical products that are encompassed by the invention include capsules, tablets, pellets, pessaries and suppositories.

The pharmaceutical products of the invention are solid when administered. Preferred solid pharmaceutical products of the invention are solid at a temperature of at least 20°C, more preferably at a temperature of at least 30°C, still more preferably at a temperature of at least 37°C (i.e. body temperature), yet more preferably at a temperature of at least 40°C.

As used herein, the term "pharmaceutical product" refers to a mixture of at least two different components. Thus 5-ALA acid or an ALA derivative on its own does not constitute a pharmaceutical product. Preferred pharmaceutical products comprise at least one pharmaceutically acceptable carrier or excipient.

As used herein the term "treatment" encompasses curative as well as prophylactic treatment.

The term "precursors" as used herein refers to precursors for 5-ALA which are converted metabolically to it and are thus essentially equivalent thereto. Thus the term "precursor" covers biological precursors for protoporphyrin in the metabolic pathway for haem biosynthesis. The term "derivatives" includes pharmaceutically acceptable salts and chemically modified agents, for example esters such as 5-ALA esters.

The use of 5-ALA and derivatives thereof (e.g. 5-ALA esters) in PDT is well known in the scientific and patent literature (see, for example, WO 2006/051269, WO 2005/092838, WO 03/011265, WO 02/09690, WO 02/10120 and US 6,034,267, the contents of which are incorporated herein by reference). All such derivatives of 5-ALA and their pharmaceutically acceptable salts are suitable for use in the methods herein described.

The 5-ALA derivatives useful in accordance with the invention may be any derivative of 5-ALA capable of forming protoporphyrin IX (PpIX) or any other photosensitiser (e.g. a PpIX derivative) *in vivo.* Typically, such derivatives will be a precursor of PpIX or of a PpIX derivative (e.g. a PpIX ester) in the biosynthetic pathway for haem and which are therefore capable of inducing an accumulation of PpIX at the site of the disease following administration *in vivo.* Suitable precursors of PpIX or PpIX derivatives include 5-ALA prodrugs which might be able to form 5-ALA *in vivo* as an intermediate in the biosynthesis of PpIX or which may be converted (e.g. enzymatically) to porphyrins without forming 5-ALA as an intermediate. 5-ALA esters, and pharmaceutically acceptable salts thereof, are among the preferred compounds for use in the methods herein described.

Esters of 5-aminolevulinic acid and N-substituted derivatives thereof are preferred photosensitisers for use in the invention. Those compounds in which the 5-amino group is unsubstituted (i.e. the ALA esters) are particularly preferred. Such compounds are generally known and described in the literature (see, for example, WO 96/28412 and WO 02/10120 to Photocure ASA, the contents of which are incorporated herein by reference).

Esters of 5-aminolevulinic acid with substituted or unsubstituted, preferably substituted, alkanols, i.e. alkyl esters or, more preferably, substituted alkyl esters, are especially preferred photosensitisers for use in the invention. Examples of such compounds include those of general formula I:

R²₂N-CH₂COCH₂-CH₂CO-OR¹ (I)

(wherein
R¹ represents a substituted or unsubstituted, preferably substituted, straight-chained, branched or cyclic alkyl group (e.g. a substituted straight-chained alkyl group); and each R² independently represents a hydrogen atom or an optionally substituted alkyl group, e.g. a group R¹) and pharmaceutically acceptable salts thereof.

As used herein, the term "alkyl", unless stated otherwise, includes any long or short chain, cyclic, straight-chained or branched aliphatic saturated or unsaturated hydrocarbon group. The unsaturated alkyl groups may be mono- or polyunsaturated and include both alkenyl and alkynyl groups. Unless stated otherwise, such groups may contain up to 40 atoms. However, alkyl groups containing up to 30, preferably up to 10, particularly preferably up to 8, especially preferably up to 6, e.g. up to 4 carbon atoms are preferred.

The substituted alkyl R¹ and R² groups may be mono or poly-substituted. Suitable substituents may be selected from hydroxy, alkoxy, acyloxy, alkoxycarbonyloxy, amino, aryl, nitro, oxo, fluoro, -SR₃, -NR³₂ and -PR³₂ groups, and each alkyl group may be optionally interrupted by one or more -O-, -NR₃-, -S-or -PR₃- groups, in which R³ is a hydrogen atom or a C₁₋₆ alkyl group).

Preferred substituted alkyl R¹ groups include those carrying one or more oxo groups, preferably straight-chained C₄₋₁₂ alkyl (e.g. C₈₋₁₀ alkyl) groups substituted by one, two or three (preferably two or three) oxo groups. Examples of such groups include 3,6-dioxa-1-octyl and 3,6,9-trioxa-1-decyl groups.

Particularly preferred for use in the invention are those compounds of formula I in which at least one R² represents a hydrogen atom. In especially preferred compounds each R² represents a hydrogen atom.

Compounds of formula I in which R¹ represents an unsubstituted alkyl group (preferably C₁₋₈ alkyl, e.g. C₁₋₆ alkyl) or more preferably an alkyl group (e.g. C₁₋₂ alkyl, especially C₁ alkyl) substituted by a substituent as hereinbefore defined (e.g. by an aryl group such as phenyl or by an alkoxy group such as methoxy) are also preferred.

Unsubstituted alkyl groups which may be used in the invention include both branched and straight-chained hydrocarbon groups. Compounds of formula I in which R¹ is a C₄₋₈, preferably a C₅₋₈, straight chain alkyl group which is branched by one or more C₁₋₆ (e.g. C₁₋₂ alkyl) groups are preferred. Representative examples of suitable unsubstituted branched alkyl groups include 2-methylpentyl, 4-methylpentyl, 1-ethylbutyl and 3,3-dimethyl-1-butyl. 4-methylpentyl is particularly preferred.

Compounds of formula I in which R¹ is a C₁₋₁₀ straight-chained alkyl group are also preferred. Representative examples of suitable unsubstituted alkyl groups include methyl, ethyl, propyl, butyl, pentyl, hexyl and octyl (e.g. n-propyl, n-butyl, n-pentyl, n-hexyl and n-octyl). Hexyl, especially n-hexyl, is a particularly preferred group. Methyl is also particularly preferred.

Particularly preferred for use in the invention are those compounds of formula I in which R¹ represents a C₁₋₂ alkyl group (preferably a C₁ alkyl group) optionally substituted by an aryl group.

Still further preferred for use in the invention are those compounds of formula I in which R¹ represents an alkyl group (e.g. C₁₋₂ alkyl, especially C₁ alkyl) substituted by an aryl group (e.g. phenyl). Preferred substituted alkyl R¹ groups which may be present in compounds of formula I include C₁₋₆ alkyl, preferably C₁₋₄ alkyl, particularly preferably C₁ or C₂ alkyl (e.g. C₁ alkyl) substituted (preferably terminally substituted) by an optionally substituted aryl group.

By an "aryl group" is meant a group which is aromatic. Preferred aryl groups comprise up to 20 carbon atoms, more preferably up to 12 carbon atoms, for example, 10 or 6 carbon atoms.

Aryl groups which may be present in the compounds of the invention may be heteroaromatic (e.g. 5-7 membered heteroaromatics) but are preferably non-heteroaromatic. By "non-heteroaromatic" is meant an aryl group having an aromatic system comprising electrons originating solely from carbon atoms. Preferred aryl groups include phenyl and napthyl, especially phenyl. In preferred compounds for use in the invention one or two aryl groups may be present, preferably one.

In a preferred aspect the invention provides the use of a photosensitiser which is a compound of formula I wherein R¹ represents an aryl substituted C₁₋₄ alkyl group (preferably C₁₋₂, e.g. C₁), preferably wherein said aryl group comprises up to 20 carbon atoms (e.g. up to 12 carbon atoms, especially 6 carbon atoms) and is itself optionally substituted, and each R² is as hereinbefore defined (e.g. each R² is hydrogen), or a pharmaceutically acceptable salt thereof in the manufacture of a medicament for use in the prevention or treatment of acne.

Aryl groups which may be present in the compounds of the invention may optionally be substituted by one or more (e.g. 1 to 5), more preferably one or two, groups (e.g. one group). Preferably the aryl group is substituted at the meta or para position, most preferably the para position. Suitable substituent groups may include haloalkyl (e.g. trifluoromethyl), alkoxy (i.e. -OR groups wherein R is preferably a C₁₋₆ alkyl group), halo (e.g. iodo, bromo, more especially chloro and fluoro), nitro and C₁₋₆ alkyl (preferably C₁₋₄ alkyl). Preferred C₁₋₆ alkyl groups include methyl, isopropyl and t-butyl, particularly methyl. Particularly preferred substituent groups include chloro and nitro. Still more preferably the aryl group is unsubstituted.

Preferred compounds for use in the invention include methyl ALA ester, ethyl ALA ester, propyl ALA ester, butyl ALA ester, pentyl ALA ester, hexyl ALA ester, octyl ALA ester, 2-methoxyethyl ALA ester, 2-methylpentyl ALA ester, 4-methylpentyl ALA ester, 1-ethylbutyl ALA ester, 3,3-dimethyl-1-butyl ALA ester, benzyl ALA ester, 4-isopropylbenzyl ALA ester, 4-methylbenzyl ALA ester, 2-methylbenzyl ALA ester, 3-methylbenzyl ALA ester, 4-[t-butyl]benzyl ALA ester, 4-[trifluoromethyl]benzyl ALA ester, 4-methoxybenzyl ALA ester, 3,4-[dichloro]benzyl ALA ester, 4-chlorobenzyl ALA ester, 4-fluorobenzyl ALA ester, 2-fluorobenzyl ALA ester, 3-fluorobenzyl ALA ester, 2,3,4,5,6-pentafluorobenzyl ALA ester, 3-nitrobenzyl ALA ester, 4-nitrobenzyl ALA ester, 2-phenylethyl ALA ester, 4-phenylbutyl ALA ester, 3-pyridinyl-methyl ALA ester, 4-diphenyl-methyl ALA ester and benzyl-5-[(1-acetyloxyethoxy)-carbonyl]amino levulinate.

Still further preferred compounds for use in the invention include methyl ALA ester, ethyl ALA ester, 2-methoxyethyl ALA ester, benzyl ALA ester, 4-isopropylbenzyl ALA ester, 4-methylbenzyl ALA ester, 2-methylbenzyl ALA ester, 3-methylbenzyl ALA ester, 4-[t-butyl]benzyl ALA ester, 4-[trifluoromethyl]benzyl ALA ester, 4-methoxybenzyl ALA ester, 3,4-[di-chloro]benzyl ALA ester, 4-chlorobenzyl ALA ester, 4-fluorobenzyl ALA ester, 2-fluorobenzyl ALA ester, 3-fluorobenzyl ALA ester, 2,3,4,5,6-pentafluorobenzyl ALA ester, 3-nitrobenzyl ALA ester, 4-nitrobenzyl ALA ester, 2-phenylethyl ALA ester, 4-phenylbutyl ALA ester, 3-pyridinyl-methyl ALA ester, 4-diphenyl-methyl ALA ester and benzyl-5-[(1-acetyloxyethoxy)-carbonyl] amino levulinate

Particularly preferred compounds for use in the invention include benzyl ALA ester, 4-isopropylbenzyl ALA ester, 4-methylbenzyl ALA ester, 2-methylbenzyl ALA ester, 3-methylbenzyl ALA ester, 4-[t-butyl]benzyl ALA ester, 4-[trifluoromethyl]benzyl ALA ester, 4-methoxybenzyl ALA ester, 3,4-[dichloro]benzyl ALA ester, 4-chlorobenzyl ALA ester, 4-fluorobenzyl ALA ester, 2-fluorobenzyl ALA ester, 3-fluorobenzyl ALA ester, 2,3,4,5,6-pentafluorobenzyl ALA ester, 3-nitrobenzyl ALA ester, 4-nitrobenzyl ALA ester, 2-phenylethyl ALA ester, 4-phenylbutyl ALA ester, 3-pyridinyl-methyl ALA ester, 4-diphenyl-methyl ALA ester and benzyl-5-[(1-acetyloxyethoxy)-carbonyl]amino levulinate.

Especially preferred compounds for use in the methods herein described include benzyl ALA ester, 4-isopropylbenzyl ALA ester and 4-methylbenzyl ALA ester, especially benzyl ALA ester. 4-Nitrobenzyl ALA ester, 4-chlorobenzyl ALA ester and benzyl ALA ester are especially preferred.

Yet more preferred compounds for use according to the invention are 5-ALA, 5-ALA methyl ester, 5-ALA hexyl ester, 5-ALA benzyl ester and physiologically acceptable salts thereof. Amongst these, 5-ALA hexyl ester and its physiologically tolerable salts are especially preferred, e.g. 5-ALA hexyl ester in the form of its HCl salt.

The compounds for use in the invention may be prepared by any conventional procedure available in the art (e.g. as described in WO 02/10120 to Photocure ASA). For example, esters of 5-ALA may be prepared by reaction of 5-ALA with the appropriate alcohol in the presence of base. Alternatively compounds for use in the invention may be available commercially (e.g. from Photocure ASA, Norway).

The compounds for use according to the method of the invention may be in the form of a free amine (e.g. -NH₂, -NHR² or -NR²R²) or preferably in the form of a physiologically acceptable salt. Such salts preferably are acid addition salts with physiologically acceptable organic or inorganic acids. Suitable acids include, for example, hydrochloric, nitric, hydrobromic, phosphoric, sulphuric, sulphonic and sulphonic acid derivatives. Particularly preferred salts are acid addition salts with sulphonic acid or sulphonic acid derivatives as described in WO 2005/092838 to PhotoCure ASA, the entire contents of which are incorporated herein by reference. Procedures for salt formation are conventional in the art.

The compounds hereinbefore described may be used for the manufacture of a solid pharmaceutical product in any conventional manner. The desired concentration of photosensitiser in the pharmaceutical products of the invention will vary depending on several factors including the nature of the compound, the nature and form of the product in which this is presented, the intended mode of administration, the nature of the cancer to be treated or diagnosed and the subject to be treated. Generally, however, the concentration of photosensitiser is conveniently in the range 1 to 50%, preferably 1 to 40%, e.g. 2 to 25%, preferably 5 to 20% by weight of the total weight of the pharmaceutical product.

Preferred pharmaceutical products for use in the invention comprise at least one pharmaceutically acceptable carrier and/or excipient. The skilled man will be able to select suitable carriers and excipients based on, for example, the route of administration chosen and the cancer to be treated or diagnosed. Representative examples of excipients and carriers that may be used in the pharmaceutical products include agar, alginic acid, ascorbic acid, amino acids, calcium salts (e.g. calcium hydrogen phosphate), ammonium salts (e.g. ammonium acetate), carbomers, carbopols, cellulose compounds and derivatives (e.g. microcrystalline cellulose, methylcellulose, ethylcellulose, hydroxyethyl cellulose, hydroxypropylcellulose), citric acid, starch compounds and derivatives (e.g. corn starch, croscaramellose, crospovidone, cyclodextrins such as beta-cyclodextrin, lactose such as anhydrous lactose or hydrous lactose, maltodextrin, mannitol), menthol, synthetic polymers (e.g. methacrylic acid copolymers), polyethylene glycol derivatives (e.g. polysorbate), potassium salts (e.g. potassium hydrogen phosphate), sodium salts (e.g. sodium carbonate), povidone, sorbitan derivatives, talcum, wax, polyethylene glycol, poloxamer, medium-chain triglycerides, glycerides of C₈₋₁₈ fatty acids (e.g. hard fat) and mixtures thereof. Miglyol® oils, which are esters of saturated coconut and palm kernel oil-derived caprylic and capric fatty acids and glycerin or propylene glycol, are particularly preferred for use in the invention. These may, for example, be used when forming liquid-containing capsules containing the photosensitising agent.

Further pharmaceutical excipients and carriers that may be used in the pharmaceutical products herein described are listed in various handbooks (e.g. D.E. Bugay and W.P. Findlay (Eds) Pharmaceutical exipients (Marcel Dekker, New York, 1999), E-M Hoepfner, A. Reng and P.C. Schmidt (Eds) Fiedler Encyclopedia of Excipients for Pharmaceuticals, Cosmetics and Related Areas (Editio Cantor, Munich, 2002) and H.P. Fielder(Ed) Lexikon der Hilfsstffe fur Pharmazie, Kosmetik und angrenzende Gebiete (Editio Cantor Aulendorf,1989)).

Penetration enhancers may have a beneficial effect in enhancing the photosensitising effect of the photosensitiser present in the pharmaceutical products of the invention. Surface penetration assisting agents, especially dialkylsulphoxides such as dimethylsulphoxide (DMSO) may therefore be included in the products. The surface penetration assisting agent may be any of the skin penetration assisting agents described in the pharmaceutical literature, e.g. chelators (e.g. EDTA), surfactants (e.g. sodium dodecyl sulfate), non-surfactants, bile salts (sodium deoxycholate) and fatty acids (e.g. oleic acid). Examples of appropriate surface penetration assisting agents include isopropanol, HPE-101 (available from Hisamitsu), DMSO and other dialkylsulphoxides, in particular n-decylmethyl sulphoxide (NDMS), dimethylsulphacetamide, dimethylfornamide (DMFA), dimethylacetamide, glycols, various pyrrolidone derivatives (Woodford et al., J. Toxicol. Cut. & Ocular Toxicology, 1986, 5: 167-177) and Azone® (Stoughton et al., Drug Dpv. Ind. Pharm. 1983, 9: 725-744) or mixtures thereof. Preferred for use in the formulations herein described are those surface penetration assisting agents which are solid at ambient temperature.

The surface penetration agent may conveniently be provided in a concentration range of 0.2 to 50 % by weight of the total weight of the pharmaceutical product in which it is present, e.g. about 10 % by weight of the total weight of the pharmaceutical product in which it is present.

Chelating agents may also have a beneficial effect in enhancing the photosensitising effect of the photosensitiser present in the pharmaceutical products of the invention. Chelating agents may, for example, be included in order to enhance the accumulation of Pp since the chelation of iron by the chelating agent prevents its incorporation into Pp to form haem by the action of the enzyme ferrochelatase, thereby leading to a build up of Pp. The photosensitising effect is therefore enhanced.

Suitable chelating agents that may be included in the pharmaceutical products of the invention include aminopolycarboxylic acids, such as any of the chelants described in the literature for metal detoxification or for the chelation of paramagnetic metal ions in magnetic resonance imaging contrast agents. Particular mention may be made of EDTA, CDTA (cyclohexane triamine tetraacetic acid), DTPA and DOTA and well known derivatives and analogues thereof. EDTA and DTPA are particularly preferred. To achieve the iron-chelating effect, desferrioxamine and other siderophores may also be used, e.g. in conjunction with aminopolycarboxylic acid chelating agents such as EDTA.

Where present, the chelating agent may conveniently be used at a concentration of 0.05 to 20%, e.g. 0.1 to 10% by weight based on the pharmaceutical product in which it is present.

The pharmaceutical products of the invention may additionally comprise an anti-cancer agent. Thus viewed from a further aspect the invention provides use of a photosensitiser which is 5-ALA or a precursor or derivative thereof (e.g. a 5-ALA ester), together with an anti-cancer agent in the manufacture of a pharmaceutical product for use in the treatment of cancer or an infection associated with cancer, wherein said pharmaceutical product is in the form of a solid.

Viewed from a still further aspect the invention provides a kit or pack containing a pharmaceutical product as hereinbefore defined, and separately an anti-cancer agent for simultaneous, separate or sequential use in a method of treating cancer or an infection associated with cancer.

Preferred anti-cancer agents present in the pharmaceutical product and kit of the invention are anti-neoplastic agents. Representative examples of anti-neoplastic agents include alkaloids (e.g. incristine, vinblastine, vinorelbine, topotecan, teniposiode, paclitaxel, etoposide and docetaxel), alkylating agents (e.g. alkyl sulfonates such as busulfan), aziridines (e.g. carboquone, ethylenimines and methylmelamines), nitrogen mustards (e.g. chlorambucil, cyclophosphamide, estramustin, ifosfamide and melphalan), nitrosurea derivatives (e.g. carmustine and lomustine), antibiotics (e.g. mitomycins, doxorubicin, daunorubicin, epirubicin and bleomycins), antimetabolites (e.g. folic acid analogues and antagonists such as methotrexate and raltitrexed), purine analogues (e.g. 6-mercaptopurine), pyrimidine analogues (e.g. tegafur, gemcitabine, fluorouracil and cytarabine), cytokines, enzymes (e.g. L-asparginase, ranpirnase), immunomodulators (e.g. interferons, immunotoxins, monoclonal antibodies), taxanes, topoisomerase inhibitors, platinum complexes (e.g. carboplatin, oxaliplatin and cisplatin) and hormonal agents (e.g. androgens, estrogens, antiestrogens) and aromatase inhibitors. Other anti-neoplastic agents for use in the invention include imiquimod, irenotecan, leucovorin, levamisole, etopisde and hydroxyurea.

Particularly referred anti-cancer agents for use in the invention include 5-fluorouracil, imiquimod, cytokines, mitomycin C, epirubicin, irenotecan, oxalipatin, leucovorin, levamisole, doxorubicin, cisplatin, etoposide, doxirubicin, methotrexate, taxanes, topoisomerase inhibitors, hydoroxyurea and vinorelbine. Yet more preferred for use as anti-cancer agents are antibiotics such as mitomycin and pyrimidine analogues such as 5-fluorouracil.

The pharmaceutical products may additionally include lubricating agents, wetting agents, preserving agents, flavouring agents and/or odour enhancers. The pharmaceutical products for use in the method of the invention may be formulated so as to provide quick, sustained or delayed release of the photosensitiser after administration to the patient by employing procedures well known in the art. Where these are intended for oral administration in treating conditions in the lower gastrointestinal tract, delayed release formulations are preferred.

Preferred pharmaceutical products of the invention do not, however, comprise a non-aqueous liquid which has a dielectic constant of less than 80 at 25°C. Particularly preferred pharmaceutical products do not comprise a non-aqueous liquid selected from alcohols, ethers, esters, poly(alkylene glycols), phospholipids, DMSO, N-vinylpyrrolidone, N, N-dimethylacetamide and mixtures thereof.

The solid pharmaceutical products used in the method of the invention may take any conventional solid form, e.g. powder, granule, pellet, tablet, pessary, suppository or capsule.

Particularly preferred solid pharmaceutical products for use in the invention comprise a photosensitiser as hereinbefore described in the form of a solid composition. Thus preferred pharmaceutical products for use in the invention are tablets, powders, granules, pellets, suppositories and pessaries. Capsules containing powder, pellet or granulate compositions are also preferred pharmaceutical products. Capsules containing semi-solid or liquid (preferably non-aqueous liquids) are also suitable for use in the invention. The capsule may be coated. Preferred coatings are those described below.

Preferred solid pharmaceutical products of the invention are in the form of a tablet, suppository, pellet, capsule or pessary. These products preferably comprise at least one photosensitiser as hereinbefore described in the form of a solid composition. Such products are themselves new and form a further aspect of the invention.

Where the product is provided in the form of pellets (e.g. tiny pills), these can be administered as such. Alternatively, the pellets may be incorporated into a tablet or capsule. Tablets or capsules comprising a plurality of pellets are particularly preferred for use in the methods herein described and form a further aspect of the invention. Similarly, where the product is provided in the form of a tablet, this may be administered as such or, alternatively, may be incorporated into capsules to provide a capsule-unit dose comprising a plurality of mini-tablets.

It is preferred that the formulations herein described, especially those adapted for oral administration, provide for delayed release of the photosensitiser, especially when these are intended for use in the treatment or diagnosis of conditions in the lower gastrointestinal tract. Delayed (e.g. sustained) release may be achieved using any of the conventional methods known and described in art such as, for example, pH-dependent systems designed to release the photosensitiser in response to a change in pH and time-dependent (or timed-release) systems designed to release the photosensitiser after a pre-determined time.

Preferably, the solid formulations herein described (e.g. tablets, capsules and pellets) may include one or more additional components that prolong the release of the active photosensitising agent. Such delayed release agents are well known in the art and may include, for example, gums such as guar gum. The desired content of such components (e.g. gums) in the solid formulation can readily be determined by those skilled in the art and may, for example, be in the range 10 to 70 weight-%, typically around 50 weight-%.

Particularly suitable delayed release agents for use in the compositions herein described are Gelucire compositions. These are inert semi-solid waxy materials which are amphiphilic in character and are available with varying physical characteristics. They are identified by their melting point/HLB value. The melting point is expressed in degrees Celsius and the HLB (Hydrophile-Lipophile Balance) is a numerical scale extending from 0 to approximately 20. Lower HLB values denote more lipophilic and hydrophobic substances, and higher values denote more hydrophilic and lipophobic substances. Gelucire compositions are generally considered to be fatty acid esters of glycerol and PEG esters or polyglycolised glycerides. The family of Gelucire compositions is characterised by a wide range of melting points of from about 33°C to about 64°C and most commonly from about 35°C to about 55°C, and by a variety of HLB values of from about 1 to about 14, most commonly from about 7 to about 14. For example, Gelucire 44/14 designates a melting point of approximately 44°C and an HLB value of about 14. The appropriate choice of melting point/HLB value of a Gelucire or a mixture of Gelucire compositions may provide the desired delivery characteristics for sustained release. Gelucire 44/14 and Gelucire 50/02 have been found to be particularly suitable for use in the invention, either alone or in combination. When used in combination, 50:50 (w/w) and 75:25 (w/w) mixtures of Gelucire 44/14 and Gelucire 50/02 have been found to be particularly effective in providing the desired delayed release characteristics.

Other methods for tailoring the release profile of the photosensitising agent include the use of additional excipients which degrade at the intended site of treatment or where diagnosis is to be performed (e.g. in the lower part of the gastrointestinal system). In this way, the photosensitiser is delivered directly to the desired point of treatment or diagnosis. For example, the photosensitising agent may be formulated with (e.g. embedded in) a matrix which degrades in the lower part of the gastrointestinal system. For example, formulations may be designed which use enteric polymers that have a relatively high threshold pH for dissolution. Examples of suitable matrix-forming agents include carbohydrates, for example disaccharides, oligosaccharides and polysaccharides. Other suitable matrix materials include alginates, amylase, celluloses, xanthan gum, tragacanth gum, starch, pectins, dextran, cyclodextrins, lactose, maltose and chitosan.

Coated solid formulations may also provide the desired delayed release characteristics whereby the coating degrades after a pre-determined period of time within the body or at the pH of the desired target site within the gastrointestinal tract. Typical coating materials to be used according the present invention include synthetic, semi-synthetic or synthetic polymers. Preferred polymers are cellulose acetate phthalate, cellulose acetate trimellitate, polyvinyl acetate phthalate, methacrylic acid copolymers such as Eudragit®, hydroxypropyl methyl cellulose phthalate, hydroxypropylmethylcellulose phthalate, pectins and pectin salts, and cross-linked polymers and copolymers, for example 2-hydroxy-ethyl methacrylate crosslinked with divinylbenzene and N,N'-bis(beta-styrene sulphonyl-4,4'-diaminobenzene.

Other formulations and methods of administration may be used to achieve not only the desired prolonged or delayed release of the photosensitising agent, but also a high and substantially homogeneous (i.e. uniform) concentration of 5-ALA or derivatives thereof in the lower part of the gastrointestinal system. When performing PDT or PDD it is preferable to cover the whole colon with the photosensitising agent. By regulating the time and place of release of the agent in the colon, the desired uniform coverage may be achieved. Suitable for use in this regard are dosage forms or dosage regimes which comprise a plurality of individual doses (e.g. tablets, capsules or a mixture of pellets) which are capable of releasing the active component at different rates and/or at different time intervals following administration. The individual doses may be contained within a single dosage form, for example a plurality of pellets, tiny pills, granules or mini-tablets may be provided within a single tablet or capsule in which the individual pellets, pills, granules or mini-tablets are capable of providing different release profiles for the active photosensitising agent. These are generally referred to as "multi-particulate systems". Alternatively, the dosage may comprise one or more (preferably several) single dose forms (e.g. one or more tablets or capsules) intended for separate or simultaneous administration in which the individual single dose forms differ in their release profiles. When treating a patient it is envisaged that two or more different doses (e.g. capsules or tablets) containing the photosensitising agent will be administered which have different release profiles. For example, when using three different capsules it is possible to target the beginning, middle and end of the colon. Due to the peristaltic movement of the colon, the different doses will travel further down the colon before releasing their content thereby assuring a better (i.e. more uniform) "coating" of the colon wall.. In the case where the clinical dose comprises more than one unit dose, the different unit doses can be administered at the same time or at different time intervals.

The different release profiles (whether from individual particulates, e.g. pellets, within a single dosage form or from a plurality of single dose forms) may be achieved by any of the means previously described, for example by altering the nature and/or concentration of any release agent, by providing a suitable coating, etc. Where a coating is used, the nature of the coating material, its thickness and/or the concentration of the components within the coating may be varied as required to obtain the desired delayed release. Where the same coating material is used to coat a plurality of pellets, tablets or capsules, delayed release may be achieved by progressively increasing the concentration of the coating agent used to coat the individual doses. When coated pellets or granules are filled into a capsule or compressed together with conventional excipients to form a tablet, the formulation is considered a multi-particulate dosage form. In these, the tablets or capsules containing coated pellets or granules can be further coated with a suitable enteric coating which may be the same or different to that used for coating of the pellets and granules.

Alternatively, a combination of rapid and slow release agents may be used to provide the desired release profile. A suitable dosage regime may, for example, comprise administration of a plurality of capsules or tablets containing different release agents. In this regard, capsules containing Miglyol have been found to be suitable for relatively rapid release of the photosensitising agent whereas those containing Gelucire provide a much slower (delayed) release. Administration of a combination of these capsules may therefore be used to provide an improved coating of the entire colon mucosa.

A preferred aspect of the present invention thus relates to an oral therapeutic or diagnostic dose of 5-ALA or a derivative thereof (e.g. a 5-ALA ester) which comprises a plurality of tablets or capsules or a mixture of pellets comprising components that are degraded in the lower part of the gastrointestinal system in which the individual tablets, capsules or pellets are degraded with kinetic profiles whereby to secure a high and homogenous distribution of 5-ALA or 5-ALA derivative in the lower part of the gastrointestinal system. The total dose may comprise several types of pellets in, for example, one capsule where the pellets are degraded with different kinetic profiles that prolong release of 5-ALA or a 5-ALA derivative. Another option is that the therapeutic or diagnostic dose comprises several single dose forms (more than one tablet or capsule) where the single dose forms have different kinetic degradation profiles.

The oral dose formulations herein described may, for example, be provided in a pack which comprises a plurality of individual doses having different release profiles. For ease of use, the individual doses (e.g. capsules) may be colour coded with different colours. Such packs also form part of the invention.

Tablets, capsules and pellets for use in the present invention may be prepared by any conventional method. Preferably, however, tablets are prepared by direct compression of a composition as hereinbefore described or by compression after granulation.

Tablets for use in the method of the invention may be coated as herein described. Particularly preferred coatings for use on tablets as well as capsules are those which are enterosoluble and gastroresistant. Such coatings render the tablet or capsule stable to stomach pH and thus the tablet/capsule only begins to release the photosensitiser contained therein after entry into the intestinal system, e.g. the colon. Representative examples of materials suitable for use as such coatings include cellulose acetate, hydroxypropylmethylcellulose, copolymers of methacryclic acid and methacrylic esters and polyvinylacetophthalate. Other suitable coatings include cellulose acetate phatalate (CAP), ethyl cellulose, dibutyl phatalate and diethyl phatalate. The Eudragit® grades of polymer which are capable of sustained release are also particularly suitable for use as coating materials. These are based on copolymers of acrylate and methacrylates with quaternary ammonium groups as functional groups as well as ethylacrylate methylmethacrylate copolymers with a neutral ester group. Such polymers are insoluble and permeable and their release profiles can be altered by varying mixing ratios and/or coating thickness. It is preferred that such coatings should not degrade in the stomach (low pH) but be degraded in the colon where the pH is generally about 6.5. Suitable Eudragit® polymers include the Eudragit® S- and L-types.

Suppositories and pessaries for use in the present invention may be prepared by any conventional method, e.g. by direct compression of a composition comprising a photosensitiser as hereinbefore described, by compression after granulation or by moulding. Preferred suppositories are adapted for insertion into the uterus, vagina or cervix.

Suppositories and pessaries may be formulated using any of the excipients and carriers mentioned above, e.g. lactose, microcrystalline cellulose or crospovidone. Water-soluble suppositories and pessaries may be made from macrogols, propylene glycols, glycerol, gelatin or mixtures thereof. Suppositories and pessaries formulated in this way preferably melt and dissolve after administration to the body and thereby release the photosensitiser contained therein. The suppositories and pessaries herein described may further contain a bioadhesive agent, for example a mucoadhesive agent, to promote adhesion and thus prolonged contract of the composition to the mucosa membranes, e.g. the vaginal epithelium.

Alternatively suppositories or pessaries may be formulated with a fat or fat-like compound, e.g. hard fat (e.g. glycerides of C₈₋₁₈ fatty acids), a mixture of hard fat and additives, fat, paraffin, glycerol and synthetic polymers. Preferred materials are hard fats which consist mainly of mixtures of the triglyceride esters of the higher fatty acids along with varying proportions of mono- and diglycerides. Examples of suitable hard fats include the range of products sold under the trade name Witepsol (e.g. Witepsol S55, Witepsol S58, Witepsol H32, Witepsol H35 and Witepsol H37). Suppositories and pessaries formulated in this way preferably melt after administration to the body and thereby release the photosensitiser contained therein. Preferred suppositories and pessaries of this kind therefore have a melting point between 30-37°C.

An advantage of the pharmaceutical products of the invention is that they are stable. In particular the photosensitisers present within the pharmaceutical products of the invention are not prone to degradation and/or decomposition. As a result, the pharmaceutical products can be stored, e.g. at room temperature and humidity, for at least 6 months, more preferably at least 12 months, still more preferably at least 24 months or more (e.g. up to 36 months).

The solid pharmaceutical products of the present invention are preferably administered orally or topically (e.g. by insertion into the vagina or rectum). The preferred route of administration will depend on a number of factors including the severity and nature of the cancer to be treated or diagnosed, the location of the cancer and the nature of the photosensitiser. When oral administration is required, the pharmaceutical product is preferably in the form of a tablet or as powder, granules or pellets contained in a capsule (e.g. a tablet). When topical application is required, the pharmaceutical product is preferably in the form of a suppository or pessary.

After administration of the pharmaceutical product containing the photosensitiser(s), the site to be treated or diagnosed is exposed to light to achieve the desired photosensitizing effect. The length of time following administration at which the light exposure takes place will depend on the nature of the pharmaceutical product, the condition to be treated or diagnosed and the form of administration. Generally, it is necessary that the photosensitiser should reach an effective tissue concentration at the site of the cancer prior to photoactivation. This can generally take in the region of from 0.5 to 24 hours (e.g. 1 to 3 hours).

In a preferred treatment or diagnosis procedure, the photosensitiser(s) is/are applied to the affected site followed by irradiation (e.g. after a period of about 3 hours). If necessary (e.g. during treatment), this procedure may be repeated, e.g. up to a further 3 times, at intervals of up to 30 days (e.g. 7-30 days). In those cases where this procedure does not lead to a satisfactory reduction in, or complete healing of, the cancer, an additional treatment may be performed several months later.

For therapeutic purposes, methods for irradiation of different areas of the body, e.g. by lamps or lasers are well known in the art (see for example Van den Bergh, Chemistry in Britain, May 1986 p. 430-439). The wavelength of light used for irradiation may be selected to achieve an efficacious photosensitizing effect. The most effective light is light in the wavelength range 300-800 nm, typically 400-700 nm where the penetration of the light is found to be relatively deep. The irradiation will in general be applied at a dose level of 10 to 100 Joules/cm² with an intensity of 20-200 mW/cm² when a laser is used or a dose of 10-100 J/cm² with an intensity of 50-150 mW/cm² when a lamp is applied. Irradiation is preferably performed for 5 to 30 minutes, preferably for 15 minutes. A single irradiation may be used or alternatively a light split dose in which the light dose is delivered in a number of fractions, e.g. a few minutes to a few hours between irradiations, may be used. Multiple irradiations may also be applied.

For diagnostic use, the area is preferably first inspected using white light. Suspicious areas are then exposed to blue light (typically ranging from 400 - 450 nm). The emitted fluorescence (635 nm) is then used to selectively detect affected cancerous tissue. The reason for selectivity is not known, but relies most likely on the higher metabolic activity in cancer cells vs. normal cells.

The methods and uses of the invention may be used to treat and/or diagnose any cancer or any infection associated with cancer. As used herein, the term "infections associated with cancer" means any infection that is positively correlated with the development of cancer. An example of such an infection is human papillomavirus (HPV) infections.

Cancers and infections associated with cancer that may be treated and/or diagnosed may be present in any part of the body (e.g. skin, mouth, throat, oesphagus, stomach, intestines, rectum, anal canal, nasopharynx, trachea, bronchi, bronchioles, urethra, urinary bladder, ovary, urethra, vagina, cervix, uterus etc).

The methods and uses of the invention are, however, particularly useful in the treatment and diagnosis of cancer of the uterus, cervix, vagina, rectum and colon. Particularly preferably the methods and uses of the invention are used for the treatment or diagnosis of cervical cancer and colon cancer. In the treatment or diagnosis of conditions in the colon (e.g. colon cancer) an enterosoluble capsule containing the photosensitising agent (e.g. the hexyl ester of 5-ALA) has been found to be particularly effective. For the treatment of cervical cancer, the use of a suppository containing the photosensitiser (e.g. the hexyl ester of 5-ALA) is preferred.

The invention will now be described in more detail by way of the following non-limiting examples and with reference to the accompanying figures.

### Example 1 - Suppository comprising 5-ALA hexyl ester

Each suppository (2 g) contains:
Hexyl 5-aminolevulinate hydrochloride (HAL HCl) 100 mg, 10 mg or 0.8 mg Disodium edetate (EDTA) 40 mg
Witepsol S 55 or S 58 qs

Suppositories were prepared by suspending HAL HCl and disodium edetate in liquid Witepsol. The mixture was filled into a suppository mould and cooled.

### Example 2 - Stability of 5-ALA hexyl ester in suppositories based on Witepsol S55

Suppositories comprising hexyl 5-aminolevulinate hydrochloride (HAL HCl) were prepared as described in Example 1. The stability of HAL HCl in suppositories based on Witepsol S55 was investigated by HPLC analysis. Stability at both room (25°C) and refrigerator (2-8°C) temperature was tested. The results are shown in Table 1 below.

**Table 1 Stability of 100 mg HAL HCl in Witepsol S 55**

| **Time** | **Temperature (°C)** | **Assay (%)*** | **SD** |
|---|---|---|---|
| 0 | N/A | 100.3 | 1.02 |
| 2 weeks | 2-8 | 92.6 | 10.21 |
| | 25 | 99.8 | 1.12 |
| 4 weeks | 2 - 8 | 98.9 | 0.73 |
| | 25 | 98.5 | 0.89 |
| 3 months | 2 - 8 | 100.0 | 0.65 |
| | 25 | 98.0 | 0.46 |

| | | | |
|---|---|---|---|
| * Assay is calculated as % of the theoretical concentration of HAL HCl in formulation | | | |

The results in Table 1 show that suppositories comprising HAL HCl based on Witepsol S55 were stable for at least 3 months both at room temperature and refrigerator temperature.

### Example 3 - Stability of 5-ALA hexyl ester in suppositories based on Witepsol S 58

Suppositories comprising hexyl 5-aminolevulinate hydrochloride (HAL HCl) were prepared as described in Example 1. The stability of HAL HCl in suppositories based on Witepsol S 58 was investigated by HPLC analysis. Stability at both room (25°C) and refrigerator (2-8°C) temperature was tested. The results are shown in Table 2 below.

**Table 2. Stability of 100 mg HAL HCl in Witepsol S 58**

| **Time** | **Temperature (°C)** | **Assay (%)*** | **SD** |
|---|---|---|---|
| 0 | N/A | 101.6 | 1.15 |
| 2 weeks | 2 - 8 | 102.2 | 1.21 |
| | 25 | 102.0 | 1.00 |
| 4 weeks | 2 - 8 | 100.1 | 1.65 |
| | 25 | 99.4 | 1.17 |
| 3 months | 2 - 8 | 103.3 | 1.02 |
| | 25 | 100.7 | 2.14 |

| | | | |
|---|---|---|---|
| * Assay is calculated as % of the theoretical concentration of HAL HCl in formulation | | | |

The results in Table 2 show that suppositories comprising HAL HCl based on Witepsol S58 were stable for at least 3 months both at room temperature and refrigerator temperature.

### Reference example

Four batches of water-containing creams containing 160 mg/g methyl 5-aminolevulinate (MAL) were placed at room temperature (25°C) for three months and analysed at different points in time for their content of MAL. A loss of 27 ± 4 % (mean ± SD) was observed at 3 months.

Although the cream experiments were performed with MAL, rather than HAL, the results show the advantage of formulating an ALA ester in a solid pharmaceutical product.

### Example 4 - Enteric-coated oral tablets comprising 5-ALA hexyl ester

Tablet cores comprising HAL HCl were prepared by mixing of the components listed below followed by direct compression:

**Each tablet core comprises:**

| | |
|---|---|
| HAL HCl | 100 mg |
| Microcrystalline cellulose | 230mg |
| Hydroxypropyl methylcellulose | 130mg |
| Povidone | 60 mg |
| Silicon dioxide | 14mg |
| Magnesium stearate | 6 mg |
| Total tablet core weight | 530 mg |

The tablet cores were coated with several layers of cellulose acetate phatalate (CAP) by use of an acetone solution of CAP. The final tablet weight was between 540 and 700 mg.

### Example 5 - Pessary

Tablet cores were prepared as described in Example 4. The cores are sprayed with a solution of:
Ethyl cellulose (2 %)
Dibutylphthalate (1%)
Alcohols (ethyl alcohol and isopropyl alcohol) (97 %)

### Example 6 - Aerosol delivery formulation comprising 5-ALA esters

HAL HCl is blended with lactose and micronized. The particle size is approximately 2-10 microns. The amount of active material is about 4 % wt. (HAL HCl). The composition is filled into capsules for use in an inhalation device. Each capsule comprises 10 mg HAL HCl. One dose is 1 to 10 capsules.

### Example 7 - Pessary comprising 5-ALA esters

A tablet core is prepared from:

| | |
|---|---|
| HAL HCl | 50 mg |
| Lactose | 100 mg |
| Starch | 40 mg |
| PVP | 50 mg |
| Magnesium stearate | 10 mg |

HAL HCl, lactose and starch are mixed for 20 minutes. An aqueous solution of PVA is added and the obtained granulate is sieved and dried at 50 degrees centigrade for 24 hours. The material is mixed with magnesium stearate and tablets are prepared. Tablet diameter is 5 mm.

The tablet cores are coated with Eudragit S 100 and diethylphatalate by spraying of an ethanol solution comprising Eudragit S 100 (10% w/v) and diethylphthalate (3% w/v).

### Example 8 - Coated tablets comprising 5-ALA ester

Tablets comprising a core, a semi-permeable layer and an enteric coating are prepared from the materials listed below using conventional tablet preparation methods.

Each tablet comprises:

**Tablet core:**

| | |
|---|---|
| 5-ALA ester salt | 100 mg |
| Avicel PH 102 | 80 mg |
| Croscaramellose | 20 mg |
| Mannitol | 40 mg |
| Polyvinyl pyrrolidone | 10 mg |
| Magnesium stearate | 3 mg |
| | |

| Semi-permeable layer: | |
|---|---|
| Ethylcellulose | 30 mg |
| Dibutyl sebacate | 8 mg |

| Enteric coating: | |
|---|---|
| Eudragit L100 | 50 mg |
| Triethyl citrate | 6 mg |

### Examples 9-12 - Coated capsule formulations comprising 5-ALA or 5-ALA esters

The following compositions were mixed at temperatures above their melting points. The mixtures were poured into capsules and banded. The capsules are then coated with a mixture of two grades of Eudragit (S and N) to achieve a pH sensitive film.

| Example No. | Composition | Amount |
|---|---|---|
| | | |
| 9 | Gelucire 44/14 | 400 mg |
| | 5-ALA or 5-ALA ester (salt) | 100 mg |
| | | |
| 10 | Gelucire 44/14 | 200 mg |
| | Gelucire 50/02 | 200 mg |
| | 5-ALA or 5-ALA ester (salt) | 100 mg |
| | | |
| 11 | Poloxamer 188 | 400 mg |
| | 5- ALA or 5-ALA ester (salt) | 100 mg |
| | | |
| 12 | Miglyol | 400 mg |
| | 5-ALA or 5-ALA ester (salt) | 100 mg |

### Example 13 - Preparation of pellets comprising 5-ALA hexyl ester HCl salt

Two different pellet formulations were prepared as follows:

**Composition of pellet formulation A:**

| | |
|---|---|
| Carbopol | 1 weight-% |
| 5-ALA hexyl ester HCl | 1 weight-% |
| Sperolac | 24 weight-% |
| Microcrystalline cellulose (Avicel PH-102) | 74 weight-% |

**Composition of pellet formulation B:**

| | |
|---|---|
| Hydroxypropylmethylcellulose (HPMC) | 1 weight-% |
| 5-ALA hexyl ester HCl | 1 weight-% |
| Sperolac | 24 weight-% |
| Microcrystalline cellulose (Avicel PH-102) | 74 weight-% |

The average pellet diameter was 1 mm.

### Example 14 - Uncoated tablets comprising 5-ALA hexyl ester HCl in pellets

| | |
|---|---|
| Pellet formulation (Example 13 B) | 800 mg |
| Microcrystalline cellulose (Avicel PH-102) | 140 mg |
| Magnesium stearate | 10 mg |

The components were mixed and tablets prepared by direct compression. Tablet diameter: 13 mm.

### Example 15 - Coated tablets (2% CAP)

Tablets were prepared according to Example 14. The tablets were coated twice and three times with a solution of cellulose acetate phthalate (CAP) (2 weight-%) in acetone. The tablets were air dried for 30 minutes and dried for 5 minutes at 80°C.

### Example 16 - Coated HMPC pellets (2% CAP)

Pellets (formulation B, Example 13) were carefully washed with a solution of CAP (2 weight-%) in acetone. Excess solvent was removed. The pellets were dried for 30 minutes at room temperature followed by 5 minutes at 80°C.

### Example 17 - Coated carbopol pellets (2% CAP)

Pellets (formulation A, Example 13) were carefully washed with a solution of CAP (2 weight-%) in acetone. Excess solvent was removed. The pellets were dried for 30 minutes at room temperature followed by 5 minutes at 80°C.

### Example 18 - Coated carbopol pellets (4% CAP)

Pellets (formulation A, Example 13) were carefully washed with a solution of CAP (4 weight-%) in acetone. Excess solvent was removed. The pellets were dried for 30 minutes at room temperature followed by 5 minutes at 80°C.

### Example 19 - Coated carbopol pellets (6% CAP)

Pellets (formulation A, Example 13) were carefully washed with a solution of CAP (6 weight-%) in acetone. Excess solvent was removed. The pellets were dried for 30 minutes at room temperature followed by 5 minutes at 80°C.

### Example 20 - Coated carbopol pellets (8% CAP)

Pellets (formulation A, Example 13) were carefully washed with a solution of CAP (8 weight-%) in acetone. Excess solvent was removed. The pellets were dried for 30 minutes at room temperature followed by 5 minutes at 80°C.

### Example 21 - Coated carbopol pellets (10% CAP)

Pellets (formulation A, Example 13) were carefully washed with a solution of CAP (10 weight-%) in acetone. Excess solvent was removed. The pellets were dried for 30 minutes at room temperature followed by 5 minutes at 80°C.

### Example 22 - Coated tablets comprising various coated pellets

**Each tablet comprises:**

| | |
|---|---|
| Uncoated pellets (Formulation Example 13A) | 200 mg |
| Coated pellets 2% CAP (from Example 17) | 220 mg |
| Coated pellets 4% CAP (from Example 18) | 240 mg |
| Coated pellets 6% CAP (from Example 19) | 133 mg |
| Coated pellets 8% CAP (from Example 20) | 122 mg |
| Coated pellets 10% CAP (from Example 21) | 104 mg |
| Microcrystalline cellulose (Avicel PH102) | 183 mg |
| Cross caramellose sodium | 11 mg |
| Magnesium stearate | 10 mg |

The components were mixed and tablets prepared by direct compression. The tablets were coated with CAP (6 weight-% in acetone) and air dried for 30 minutes and dried for 5 minutes at 80°C. Tablet diameter: 13 mm.

### Example 23 - Coated tablets from uncoated pellets and 5-ALA hexyl ester HCl.

**Each tablet comprises:**

| | |
|---|---|
| Uncoated pellets (Formulation Example 13A) | 1000mg |
| 5-ALA hexyl ester HCl | 50 mg |
| Microcrystalline cellulose (Avicel PH-102) | 210 mg |
| Cross caramellose sodium | 23 mg |
| Magnesium stearate | 25 mg |

The components were mixed and tablets prepared by direct compression. The tablets were coated with CAP (8 weight-% in acetone) and air dried for 30 minutes and dried for 5 minutes at 80°C. Tablet diameter: 13 mm.

### Example 24 - Coated gelatine capsules comprising uncoated pellets

Uncoated pellets (Formulation Example 13A) (273 mg) were filled into a hard gelatine capsule. The size of the capsule was 17 mm, diameter 6 mm. The gelatine capsule was carefully coated twice with CAP (4% solution in acetone). The capsule product was air-dried for 30 minutes followed by drying at 80°C for 5 minutes.

### Example 25 - Coated gelatine capsule comprising 5-ALA hexyl ester HCl

5-ALA hexyl ester HCl (237 mg) was filled into a hard gelatine capsule. The capsule was carefully coated twice with CAP (10% solution in acetone) and dried as described in Example 24.

### Example 26 - Uncoated gelatine capsule comprising two types of coated pellets

| | |
|---|---|
| Coated pellets 2% CAP (from Example 17) | 278 mg |
| Coated pellets 8% CAP (from Example 20) | 376 mg |

The pellets were mixed and filled into a hard gelatine capsule and dried as described in Example 24.

### Example 27 - Chitosan tablets comprising 5-ALA hexyl ester HCl

| | |
|---|---|
| Chitosan (medium molecular weight) | 800 mg |
| Microcrystalline cellulose (Avicel PH-102) | 300 mg |
| 5-ALA hexyl ester HCl | 50 mg |
| Magnesium stearate | 17 mg |
| Silica colloidal (anhydrous) | 5 mg |

The components were mixed and tablets prepared by direct compression. Tablet diameter: 13 mm.

### Example 28 - Chitosan tablets comprising 5-ALA hexyl ester HCl

| | |
|---|---|
| Chitosan (medium molecular weight) | 506 mg |
| Microcrystalline cellulose (Avicel PH-102) | 580 mg |
| 5-ALA hexyl ester HCl | 103 mg |
| Croscaramellose sodium | 10 mg |

The components were mixed and tablets prepared by direct compression. Tablet diameter: 13 mm.

### Example 29 - Chitosan tablets coated with Eudragit®

Tablets were prepared according to Example 27. The tablets were coated twice with a dispersion of Eudragit® (Eudragit® RS30D). The tablets were air dried for 30 minutes and then dried for 5 minutes at 80°C.

### Example 30 - Chitosan tablets coated with CAP

Tablets were prepared according to Example 28. The tablets were coated twice with CAP (6 weight-% in acetone). The tablets were air dried for 30 minutes and dried for 5 minutes at 80°C.

### Example 31 - Eudragit® coated pellets comprising 5-ALA hexyl ester

Pellets (from Example 13B) were coated with Eudragit® (Eudragit RS30D dispersion). The pellets were air dried for 30 minutes and dried for 15 minutes at 80°C.

### Example 32 - Eudragit® (1.0%) coated pellets comprising 5-ALA hexyl ester

Pellets (from Example 13B) were coated with Eudragit® (Eudragit® S100, 1.0 weight-% in acetone). The pellets were air dried for 30 minutes and dried for 15 minutes at 80°C.

### Example 33 - Eudragit® (2.5%) coated pellets comprising 5-ALA hexyl ester

Pellets (from Example 13B) were coated with Eudragit® (Eudragit® S100, 2.5 weight-% in acetone). The pellets were air dried for 30 minutes and dried for 15 minutes at 80°C.

### Example 34 - Eudragit® (2.5%) coated pellets comprising 5-ALA hexyl ester

Pellets (from Example 13B) were coated with Eudragit® (Eudragit® S100, 2.5 weight-% in acetone). The pellets were air dried for 30 minutes and dried for 15 minutes at 80°C.

### Example 35 - Tablets comprising various coated pellets comprising 5-ALA hexyl ester HCl

**Each tablet comprises:**

| | |
|---|---|
| Pellets 1 % Eudragit S-100 (from Example 32) | 132 mg |
| Pellets 2.5 % Eudragit S-100 (from Example 33) | 190 mg |
| Pellets 5 % Eudragit S-100 (from Example 34) | 164 mg |
| Microcrystalline cellulose (Avicel PH-102) | 130 mg |
| Magnesium stearate | 10 mg |

The components were mixed and tablets prepared by direct compression. Tablet diameter: 13 mm.

### Example 36 - Coated tablets comprising various coated pellets comprising 5-ALA hexyl ester HCl

Tablets were prepared according to Example 35. The tablets were coated with Eudragit S-100 (3 weight-% Eudragit® and 1% triethyl citrate in acetone). The tablets were air dried for 30 minutes and dried for 5 minutes at 80°C.

### Example 37 - Enterosoluble capsules for colon release.

Size 1 HPMC capsules were coated with Eudragit L 30 D-55, Eudragit FS 30 D and triethyl citrate. The capsules contained 100 mg of hexyl 5-aminolevulinate HCl (HAL-HCl) and 300 mg of excipient(s). These included Poloxamer 188, Gelucire 44/14, Gelucire mixture (44/14: 50/02 = 50:50 w/w) and Miglyol 812 N. The excipients were included to influence the drug release into the colon after dissolution of the capsule. The capsules were coated with an enteric coating.

### Example 38 - Stability Indication

In order to get an indication of the stability of HAL in the presence of various excipients, a stress-study at 80°C was performed. 100 mg HAL HCl + 300 mg excipient (Poloxamer 188, Gelucire 44/14, Gelucire mixture (44/14: 50/02 = 50:50 w/w) and Miglyol 812 N) was mixed and the result after 20 hours was analysed by HPLC. The level of each impurity was calculated relative to a pyrazine standard. The results are given in the table below. It can be seen that hardly any impurities were detectable when Miglyol was used as an excipient, whereas the other excipients resulted in a higher number and higher levels of impurities. The sample containing Miglyol contained lower levels of impurities than the HAL HCl sample itself.

| | | | | | Gelucire 44/14 & 50/02 |
|---|---|---|---|---|---|
| Impurity* | HAL-HCl | Miglyol | Poloxamer 188 | Gelucire 44/14 | |
| 0.8 | ** | | 0.04% | 0.07% | 0.04% |
| 0.88 | 0.46% | | | 0.44% | 0.27% |
| 0.93 | | | 0.17 | | |
| Pyrazine | 1.77% | 0.70% | 1.73% | 2.89% | 1.75% |
| 1.37 | 0.03% | | 0.53% | 0.91% | 0.64% |
| 1.49 | | | 0.02% | 0.04% | 0.03% |
| 1.7 | | | | 0.01% | 0.01% |
| 1.74 | | | 0.43% | 0.21% | 0.12% |
| 1.91 | 0.35% | | 0.26% | | 0.23% |
| 1.96 | | 0.06% | 0.05% | 0.15% | 0.04% |
| 2.06 | 0.16% | | 0.06% | | |

| | | | | | |
|---|---|---|---|---|---|
| *As determined by their relative retention time (in minutes) on HPLC. **An empty entry indicates no detectable levels | | | | | |

### Example 39 - Stability study

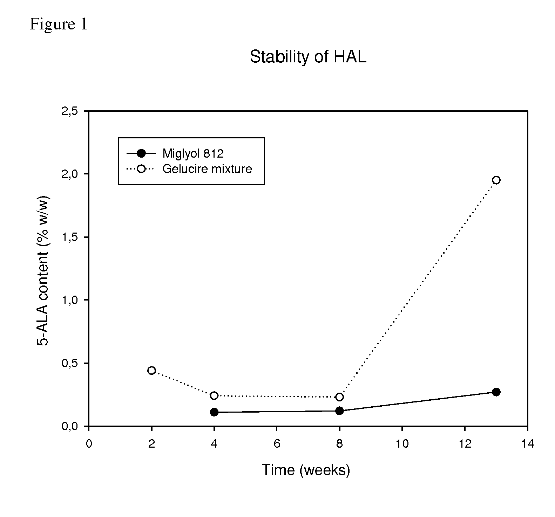

Capsules containing 100 mg HAL HCl + 300 mg excipient (Gelucire mixture (44/14: 50/02 = 50:50 w/w) or Miglyol 812 N) were prepared as described in Example 37 and monitored for stability at 25°C/60% RH. To monitor stability, the concentration of 5-aminolevulinic acid (5-ALA) (formed on hydrolysis of HAL) was used as a stability indicator. The results are shown in Figure 1 which shows the liberation of 5-ALA from HAL as a result of hydrolysis. It can be seen that Miglyol 812N proved to give the most stable product with hardly any increase in the 5-ALA values. For the Gelucire mixture an increase in 5-ALA was seen after 3 months at 25°C/60 % RH. A corresponding increase in 5-ALA was also seen for Poloxamer 188 (not shown). This excipient also resulted in the formation of significant amounts of two unknown pyrazinic impurities.

### Example 40 - Dissolution studies

Capsules were coated as described in Example 37 and filled with 100 mg HAL HCl mixed with 300 mg of excipient (either Miglyol or mixed Gelucire (44/14:50/02 = 50:50 w/w)) and banded. These were used in *in-vitro* dissolution studies using a type 2 USP dissolution apparatus (with paddles) according to Ph. Eur. 2.9.3. The capsules were firstly immersed in 0.1 M HCl for 1 hr (to reflect the acidic conditions in the stomach) and then transferred to phosphate buffer (pH 6.5). Initial studies indicated that the addition of 2% sodium lauryl sulphate in the dissolution media was required as both formulations are based on fatty, hydrophobic materials. Samples were drawn and analysed for HAL at different time-points.

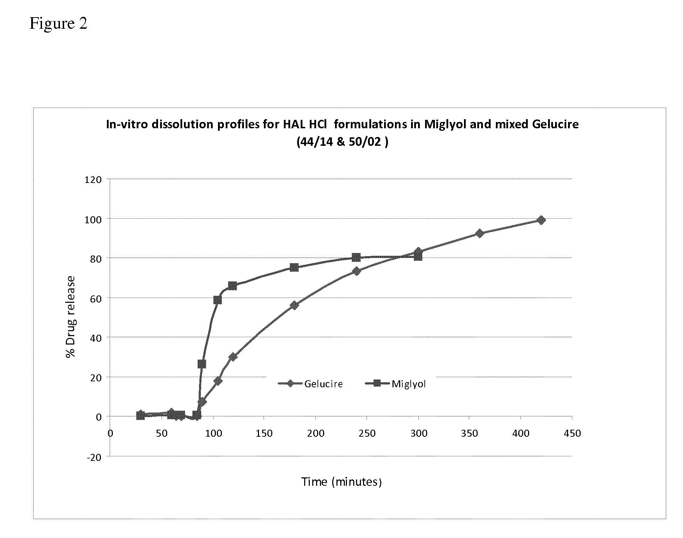

Figure 2 shows dissolution profiles for Miglyol and mixed Gelucire formulations (44/14:50/02 = 50:50 w/w). This indicates that no HAL was released in 0.1 M HCl. After transfer to phosphate buffer (pH 6.5), HAL was released faster from the Miglyol formulation as compared to the mixed Gelucire formulation, which resulted in a more prolonged release.

### Example 41 - Suppositories for the cervix

A number of suppository batches with hard fats - Witepsol H32 (mp 31-33°C), H35 (mp 33.5-35.5°C) and H37 (mp 36-38°C) - were manufactured by dissolving 200 mg HAL-HCl in 1.8 g of melted fat followed by moulding (see Example 1).

Stability testing (at 5°C and 25°C) indicated no particular stability problems - see Examples 2 and 3.

A dissolution study was performed (Ph. Eur. 2.9.3, basket apparatus) for suppositories made from Witepsol H35 and Witepsol H32 each containing 100 mg HAL HCl. Phosphate buffer (pH 4.0) at 37°C was used as dissolution media and the released drug was analyzed with HPLC. The study showed that Witepsol H32 suppositories gave a fast and nearly complete release of HAL within 1 hour unlike Witepsol H35 suppositories which released only 6 % HAL within 8 hours. Witepsol bases with higher melting points such as H37 proved to be unsuitable with respect to drug release. The difference in dissolution rate is probably due to the different melting points of the hard fats.

### Example 42 - Tablets comprising 5-ALA benzyl ester HCl

| | |
|---|---|
| Microcrystalline cellulose (Avicel PH-102) | 380 mg |
| Lactose monohydrate | 340 mg |
| 5-ALA benzyl ester HCl | 70 mg |
| Magnesium stearate | 10 mg |

The components were mixed and tablets prepared by direct compression. Tablet diameter: 13 mm.

### Example 43 - Tablets comprising 5-ALA benzyl ester coated with Eudragit®

Tablets were prepared according to Example 42. The tablets were coated with an acetone solution of Eudragit S-100 (6 %) and triethyl citrate (1%) and dried.

### Example 44 - Tablets comprising 5-ALA methyl ester HCl

| | |
|---|---|
| Microcrystalline cellulose (Avicel PH-102) | 266 mg |
| Lactose monohydrate | 280 mg |
| 5-ALA methyl ester HCl | 200 mg |
| Magnesium stearate | 10 mg |
| Crosscaramellose sodium | 15 mg |

The components were mixed and tablets prepared by direct compression. Tablet diameter: 13 mm.

### Example 45 - Tablets comprising 5-ALA methyl ester coated with Eudragit®

Tablets were prepared according to Example 44. The tablets were coated with an acetone solution of Eudragit S-100 (6 %) and triethyl citrate (1%) and dried.

### Example 46 - Tablets comprising 5-ALA HCl and pectin

| | |
|---|---|
| Microcrystalline cellulose (Avicel PH-102) | 215 mg |
| Pectin from citrus fruits | 210mg |
| Lactose monohydrate | 116 mg |
| 5-ALA HCl | 190 mg |
| Magnesium stearate | 10 mg |
| Crosscaramellose sodium | 15 mg |

The components were mixed and tablets prepared by direct compression. Tablet diameter: 13 mm.

### Example 47 - Tablets comprising 5-ALA and pectin coated with Eudragit®

Tablets were prepared according to Example 46. The tablets were coated with an acetone solution of Eudragit S-100 (6 %) and triethyl citrate (1%) and dried..

### Example 48 - Coated capsules comprising 5-ALA methyl ester

5-ALA methyl ester HCl (90 mg) was filled into a hard gelatine capsule and the gelatine capsule was coated with an acetone solution of Eudragit® S-100 (6%) and triethyl citrate (1%) and dried.
Capsule size: length: 17 mm, diameter: 6 mm.

### Example 49 - Stability of pellets comprising 5-ALA hexyl ester

Pellets comprising 5-ALA hexyl ester (from Example 13, formulation A and B) were kept in an open container in a climate cabinet for approx. 3 weeks at 40°C and 70% relative humidity.
HPLC analyses did not show any increased degradation of 5-ALA hexyl ester as a result of high temperature and high humidity.

### Example 50 - Sustained release of 5-ALA hexyl ester HCl from pellets

Pellets comprising 5-ALA hexyl ester (from Example 13, formulation A and B) (1.0 gram) were suspended in water (10 ml) and kept for several hours at 37°C. The aqueous solution was analysed for 5-ALA hexyl ester over time. The release of 5-ALA hexyl ester was less than 10% during several hours.

### Example 51 - Tablets comprising 5-ALA methyl ester and DMSO

DMSO (200 mg) was mixed with microcrystalline cellulose (500 mg) to obtain a powder (DMSO/MCC powder).

| | |
|---|---|
| DMSO/MCC powder | 700 mg |
| 5-ALA methyl ester HCl | 25 mg |
| Magnesium stearate | 10 mg |
| Crosscaramellose sodium | 15 mg |

The components were mixed and tablets prepared by direct compression. Tablet diameter: 13 mm.

### Example 52 - Coated tablets comprising 5-ALA methyl ester and DMSO

Tablets were prepared according to Example 51. The tablets were coated with an acetone solution of Eudragit S-100 (6 %) and triethyl citrate (1%) and dried..

### Example 53 - Capsule comprising 5-ALA and DMSO

DMSO (19 mg) was mixed with microcrystalline cellulose (72 mg) and 5-ALA HCl (9 mg) to obtain a powder. The powder was filled into a gelatine capsule.

### Example 54 - Coated capsule comprising 5-ALA and DMSO

A capsule was prepared according to Example 53. The capsule was coated with an acetone solution of Eudragit S-100 (6 %) and triethyl citrate (1%) and dried..

### Further embodiments of the invention are described below:

1. The use of a photosensitiser which is 5-ALA or a precursor or derivative thereof (e.g. an ALA ester), in the manufacture of a pharmaceutical product for use in the photodynamic treatment or diagnosis (e.g. treatment) of cancer, an infection associated with cancer, or in the treatment or diagnosis of a non-cancerous condition, wherein said pharmaceutical product is in the form of a solid.
2. Use according to embodiment 1 wherein said product is for use in the photodynamic treatment or diagnosis of a cancerous or non-cancerous condition in the lower part of the gastrointestinal system, e.g. for the treatment or diagnosis of colorectal cancer.
3. Use according to embodiment 1 wherein said product is for use in the photodynamic treatment or diagnosis of a cancerous or non-cancerous condition in the female reproductive system, e.g. for the treatment or diagnosis of cervical cancer.
4. Use according to any one of embodiments 1 to 3, wherein said photosensitiser is a 5-ALA ester or a pharmaceutically acceptable salt thereof.
5. Use according to any preceding embodiment, wherein said photosensitiser is a compound of general formula I:

   R²₂N-CH₂COCH₂-CH₂CO-OR¹ (I)

   (wherein
   R¹ represents a substituted or unsubstituted, preferably substituted, straight-chained, branched or cyclic alkyl group; and
   each R² independently represents a hydrogen atom or an optionally substituted alkyl group, e.g. a group R¹) or a pharmaceutically acceptable salt thereof.
6. Use according to embodiment 5, wherein in formula I each R² represents a hydrogen atom.
7. Use according to embodiment 5 or 6, wherein in formula I, R¹ represents an unsubstituted alkyl group (preferably C₁₋₈ alkyl, e.g. C₁₋₆ alkyl) or an alkyl group (e.g. C₁₋₂ alkyl, especially C₁ alkyl) substituted by an aryl group.
8. Use according to embodiment 5 or 6, wherein in formula I, R¹ represents a C₁₋₂ alkyl group (preferably a C₁ alkyl group) optionally substituted by an aryl group.
9. Use according to embodiment 8, wherein said compound is selected from methyl ALA ester, benzyl ALA ester or substituted benzyl ALA ester.
10. Use according to any one of embodiments 5 to 7, wherein in formula I, R¹ represents an alkyl group (e.g. C₁₋₂ alkyl, especially C₁) substituted by an aryl group (e.g. phenyl).
11. Use according to embodiment 5, wherein said compound is selected from benzyl ALA ester, 4-isopropylbenzyl ALA ester, 4-methylbenzyl ALA ester, 2-methylbenzyl ALA ester, 3-methylbenzyl ALA ester, 4-[t-butyl]benzyl ALA ester, 4-[trifluoromethyl]benzyl ALA ester, 4-methoxybenzyl ALA ester, 3,4-[dichloro]benzyl ALA ester, 4-chlorobenzyl ALA ester, 4-fluorobenzyl ALA ester, 2-fluorobenzyl ALA ester, 3-fluorobenzyl ALA ester, 2,3,4,5,6-pentafluorobenzyl ALA ester, 3-nitrobenzyl ALA ester, 4-nitrobenzyl ALA ester, 2-phenylethyl ALA ester, 4-phenylbutyl ALA ester, 3-pyridinyl-methyl ALA ester, 4-diphenyl-methyl ALA ester and benzyl-5-[(1-acetyloxyethoxy)-carbonyl]amino levulinate.
12. Use according to embodiment 5, wherein said compound is benzyl ALA ester.
13. Use according to any one of embodiments 5 to 7, wherein in formula I, R¹ represents an unsubstituted alkyl group (preferably C₁₋₈ alkyl, e.g. C₁₋₆ alkyl).
14. Use according to embodiment 5, wherein said compound is selected from methyl ALA ester, ethyl ALA ester, propyl ALA ester, butyl ALA ester, pentyl ALA ester, hexyl ALA ester, octyl ALA ester, 2-methylpentyl ALA ester, 4-methylpentyl ALA ester, 1-ethylbutyl ALA ester, 3,3-dimethyl-1-butyl ALA ester.
15. Use according to embodiment 5, wherein said compound is selected from methyl ALA ester, hexyl ALA ester and 4-methylpentyl ALA ester (preferably methyl ALA ester).
16. Use a according to embodiment 5, wherein said compound is hexyl ALA ester or a pharmaceutically acceptable salt thereof, e.g. the hydrochloride salt.
17. Use according to any preceding embodiment, wherein said solid pharmaceutical product is administered orally.
18. Use according to any preceding embodiment, wherein said solid pharmaceutical product is provided in the form of a tablet.
19. Use according to any one of embodiments 1 to 16, wherein said solid pharmaceutical product is administered topically.
20. Use according to any one of embodiments 1 to 16 or 19, wherein said solid pharmaceutical product is provided in the form of a suppository or pessary.
21. Use according to any preceding embodiment, wherein said pharmaceutical product is for the treatment or diagnosis of cancer of the uterus, cervix, vagina, rectum or colon.
22. Use according to any one of embodiments 1 to 20, wherein said infection associated with cancer is caused by the human papillomavirus.
23. Use of a photosensitiser which is 5-ALA or a precursor or derivative thereof, together with an anti-cancer agent in the manufacture of a pharmaceutical product for use in the photodynamic treatment of cancer or an infection associated with cancer, wherein said pharmaceutical product is in the form of a solid.
24. A kit or pack containing a pharmaceutical product as defined in any one of embodiments 1 to 20, and separately an anti-cancer agent for simultaneous, separate or sequential use in a photodynamic method of treating cancer or an infection associated with cancer.
25. A photodynamic method of treatment or diagnosis of cancer or an infection associated with cancer, said method comprising the steps of:
   (a) administering to an animal a pharmaceutical product as defined in any one of embodiments 1 to 20;
   (b) optionally waiting for a time period necessary for the photosensitiser to achieve an effective tissue concentration at the desired site; and
   (c) photoactivating the photosenstiser.
26. A photodynamic method of diagnosing cancer or an infection associated with cancer in an animal pre-administered with a pharmaceutical product as defined in any one of embodiments 1 to 20, said method comprising:
   (i) optionally waiting for a time period necessary for the photosensitiser to achieve an effective tissue concentration at the desired site; and
   (ii) photoactivating the photosenstiser.
27. The use of a photosensitiser which is 5-ALA or a precursor or derivative thereof (e.g. an ALA ester), in the manufacture of a pharmaceutical product for use in a method of surgery, wherein said pharmaceutical product is in the form of a solid.
28. A solid pharmaceutical product comprising a photosensitiser which is 5-ALA or a precursor or derivative thereof (e.g. a 5-ALA ester) and at least one pharmaceutically acceptable carrier or excipient, wherein said pharmaceutical product is provided in the form of a suppository, pessary, tablet, pellet or capsule.
29. A product according to embodiment 28, wherein said photosensitiser is incorporated in a plurality of pellets, granules, pills or mini-tablets which are provided within a tablet or capsule.
30. A product according to embodiment 28 or 29 which is provided in the form of a capsule, tablet or pellet which is capable of delayed release of said photosensitiser.
31. A solid pharmaceutical product according to any one of embodiments 27 to 30 for use in medicine, preferably for use in the photodynamic treatment or diagnosis (e.g. treatment) of cancer, an infection associated with cancer, or in the treatment or diagnosis of a non-cancerous condition.

## Claims

1. A solid pharmaceutical product comprising a photosensitiser which is a 5-ALA ester of general formula I, or a pharmaceutically acceptable salt thereof:
R²₂N-CH₂COCH₂-CH₂CO-OR¹ (I)
wherein
R¹ represents a substituted or unsubstituted, straight-chained, branched or cyclic alkyl group; and
each R² independently represents a hydrogen atom;
and at least one pharmaceutically acceptable carrier or excipient which is a hard fat or a mixture of hard fat and additives;
wherein said pharmaceutical product is provided in the form of a suppository.

2. A solid pharmaceutical product as claimed in claim 1 wherein said product is adapted for insertion into the uterus, vagina or cervix, preferably the vagina.

3. A solid pharmaceutical product as claimed in claim 1 or claim 2 wherein said product has a melting point between 30 and 37°C.

4. A solid pharmaceutical product as claimed in any one of the preceding claims wherein said hard fat or said mixture of hard fat and additives has a melting point between 31 and 33°C.

5. A solid pharmaceutical product as claimed in any one of the preceding claims which comprises a hard fat, preferably wherein said hard fat is Witepsol H 32.

6. A solid pharmaceutical product as claimed in any one of claims 1 to 4 which comprises a mixture of hard fat and additives, preferably wherein said mixture of hard fat and additives is Witepsol S 58.

7. A solid pharmaceutical product as claimed in any one of the preceding claims wherein in formula I, R¹ represents an unsubstituted alkyl group (preferably C₁₋₈ alkyl, e.g. C₁₋₆ alkyl) or an alkyl group (e.g. C₁₋₂ alkyl, especially C₁ alkyl) substituted by an aryl group.

8. A solid pharmaceutical product as claimed in any one of the preceding claims, wherein said photosensitiser is hexyl ALA ester or a pharmaceutically acceptable salt thereof, e.g. the hydrochloride salt.

9. A solid pharmaceutical product as claimed in any one of the preceding claims consisting of said photosensitiser and either a hard fat or a mixture of hard fat and additives.

10. A solid pharmaceutical product as claimed in any one of the preceding claims wherein said photosensitiser is present at a concentration in the range 1-50%, preferably 1-40%, e.g. 2 to 25%, preferably 5-20% by weight of the total weight of the pharmaceutical product.

11. A solid pharmaceutical product as claimed in any one of claims 1 to 10 for use in the photodynamic treatment of cancer, an infection associated with cancer, or in the treatment of a non-cancerous condition, wherein said pharmaceutical product is provided in the form of a suppository.

12. A solid pharmaceutical product as claimed in claim 11 for use in the photodynamic treatment of a cancerous or non-cancerous condition in the female reproductive system.

13. A solid pharmaceutical product as claimed in claim 12 for use in the photodynamic treatment of cervical cancer.

14. A solid pharmaceutical product as claimed in claim 11 for use in the treatment of an infection associated with cancer, preferably wherein said infection associated with cancer is caused by the human papillomavirus.

15. A solid pharmaceutical product for use as claimed in claim 14 wherein said infection is present in the vagina, cervix or uterus.
